# EUROPÄISCHE PATENTSCHRIFT

(11) **EP 0 752 246 B1**
(45) Veröffentlichungstag und Bekanntmachung des Hinweises auf die Patenterteilung: **13.03.2002**
(21) Anmeldenummer: 96109915.7
(22) Anmeldetag: 20.06.1996
(51) Int. Cl.: A61K 31/40, A61K 31/135, A61K 31/165, A61P 1/00

(54) **Verwendung von N-Methyl-N-[(1S)-1-phenyl-2-((3S)-3-hydroxypyrrolidin-1-yl)-ethyl]-2,2-diphenyl-acetamid Hydrochlorid zur Herstellung von Arzneimitteln zur Behandlung von entzündlichen Darmerkrankungen**
Use of N-methyl-N-[(1S)-1-phenyl-2-((3S)-3-hydroxypyrrolidin-1-yl)-ethyl]-2,2-diphenyl acetamide hydrochloride for the preparation of a medicament for the treatment of inflammatory diseases of the intestine
Utilisation de N-Méthyl-N-[(1S)-1-phényl-2-((3S)-3-dyhroxypyrrolidine-1-yl)-éthyl]-2,2-diphényl acetamide chlorhydrique pour la préparation des médicaments pour le traitement des maladies inflammatoires de l'intestin

(30) Priorität: 28.06.1995 DE 19523502
(43) Veröffentlichungstag der Anmeldung: 08.01.1997
(73) Patentinhaber: MERCK PATENT GmbH, 64293 Darmstadt (DE)
(72) Erfinder: Barber, Andrew, Dr., 64331 Weiterstadt (DE); Seyfried, Christoph, Dr., 64342 Seeheim (DE); Bartoszyk, Gerd, 64331 Weiterstadt (DE); Gottschlich, Rudolf, Dr., 64354 Reinheim (DE)

(56) Entgegenhaltungen:
- EP-A- 0 569 802
- DRUGS EXP. CLIN. RES., Bd. 21, Nr. 5, 1995, Seiten 171-4, XP002015623 GOTTSCHLICH R.: "The peripherally acting kappa opiate agonist EMD 61753 and analogs: opoid activity versus peripheral selectivity"
- CHIRALITY, Bd. 6, Nr. 8, 1994, Seiten 685-9, XP002015624 GOTTSCHLICH, R. ET. AL.: "K-opioid activity of the four stereoisomers of the peripherally selective kappa-agonists, EMD 60400 and EMD 61753"
- BR. J. PHARMACOL., Bd. 113, Nr. 4, 1994, Seiten 1317-27, XP002015625 BARBER A. ET. AL.: "A pharmacological profile of the novel, peripherally-selective kappa-opoid receptor agonist, EMD 61753"

## Beschreibung

Die Erfindungen betrifft die Verwendung der Verbindung N-Methyl-N-[(1S)-1-phenyl-2-((3S)-3-hydroxypyrrolidin-1-yl)-ethyl]-2,2-diphenyl-acetamid, Hydrochlorid (EMD 61753) zur Herstellung von Arzneimittelformulierungen zur Behandlung von entzündlichen Darmerkrankungen.

Verbindungen mit ähnlicher Strukturformel und geeignete Verfahren zu deren Herstellung sind in der Offenlegungsschrift DE 40 34 785 beschrieben.

Entzündliche Darmerkrankungen führen häufig zu Dickdarmschmerzen, Verdauungsstörungen und im schlimmsten Fall zu einem Darmverschluß. Letzterer ist mit kolikartigen Schmerzen infolge eines heftigen Kontraktionsreizes, Stuhl- und Windverhalten, Erbrechen und mit zunehmender Dauer des Zustandes Dehydratation, Abwehrspannung des Abdomens und schließlich einem Schock verbunden.

Funktionelle Darmerkrankungen werden auf verschiedenste Ursachen zurückgeführt; unter anderem auf eine Abnormität der Kontraktionsfähigkeit der glatten Darmmuskeln und der gastrointestinalen motorischen Aktivität. Eine übermäßige Kontraktionsaktivität und eine veränderte Koordination der motorischen Aktivität kann Schmerzen hervorrufen durch die Aktivierung des Mechano-Rezeptors und durch Transportabnormitäten, welche zu Dehnungen des Darms führen. Diese Ursachen wurden bisher ebenso zur Erklärung von Brustschmerzen, die nicht vom Herzen herrühren, angenommen, wie auch zur Erklärung der Schmerzen des gereizten Darmsyndroms oder einer nicht mit einem Ulkus verbundenen Dyspepsie. Indessen wurde dieser Zusammenhang weiter gestützt durch 24-stündige Aufzeich-nungen der motorischen Speiseröhren- und Gastroduodenalfunktion von Patienten, die unter nicht vom Herzen herrührenden Brustschmerzen und einer nicht mit einem Ulkus verbundenen Dyspepsie litten (Katz, P. O. et. al. Ann. Intern. Med. (1987) 106, 593-7). Motorische Abnormitäten können bei normalen Kontroll-personen ohne Symptome auftreten, können aber auch verschwinden, wodurch eine zeitliche Korrelation mit den Symptomen des Patienten aufgezeigt werden können (Fefer, L. et. al. Gastroenterology (1992) 102: A447 (Abstract).

Die Behandlung der motorischen Abnormitäten mit verschiedensten therapeutisch wirksamen Agenzien, wie beispielsweise mit Mitteln, die die Bewegungen des Magen-Darmtraktes fördern, mit Anticholinergika oder Calciumkanal- und Cholecystokininantagonisten, sind in den meisten Fällen wirksam in der Korrektur der motorischen Abnormitäten, sie verbessern jedoch nicht immer die Symptome der Patienten.

Es war daher Aufgabe der Erfindung, pharmazeutisch wirksame Verbindungen zur Verfügung zu stellen, die in der Behandlung entzündlicher Darmerkrankungen einsetzbar und wirksam sind, die gleichzeitig die mit dieser Erkrankung verbundenen Schmerzen lindern und im akuten Fall eines durch die entzündliche Darmerkrankung drohenden bzw. hervorgerufenen Darmverschlußes die Motorik des Darms wieder normalisieren oder wieder in Gang setzen, ohne spürbare Nebenwirkungen hervor-zurufen. Gleichzeitig war es Aufgabe der Erfindung, pharmazeutisch wirksame Verbindungen zur Verfügung zu stellen, die auf eine normale Darmperistaltik keine Auswirkungen haben, jedoch die Ausheilung der entzündlichen Darmerkrankung mit bewirken.

Es wurde nun gefunden, daß sich die Verbindung EMD 61753 als Arzneimittel zur Behandlung entzündlicher Darmerkrankungen in ganz besonderer Weise eignet.

Die Verbindung N-Methyl-N-[(1S)-1-phenyl-2-((3S)-3-hydroxypyrrolidin-1-yl)-ethyl]-2,2-diphenyl-acetamid, Hydrochlorid ist in der EP 0 569 802 sowie von R. Gottschlich et al. in Drugs Exp. Clin. Res. 21, 171-174 (1995) offenbart.
A. Barber et al. beschreibt in Br. J. Pharmacol. 113, 1317-1327 (1994) die analgetische Wirkung der Verbindung bei hyperalgetischem Druckschmerz sowie neurogener Entzündung und entzündungsbedingten Schmerzen.

Gegenstand der Erfindung ist somit die Verwendung von EMD 61753 zur Herstellung von Arzneimitteln zur Behandlung von entzündlichen Darmerkrankungen.

EMD 61753 zeigt besonders gute analgetische Wirkungen. In diesem Zusammenhang antagonisiert es insbesondere entzündungsbedingte Hyperalgesien, ist aber auch wirksam in der Bekämpfung des eigentlichen Entzündungsgeschehens, so daß es ein breites Wirkungsspektrum aufweist.

Versuche haben gezeigt, daß die erfindungsgemäße Verbindung im "Writhing Test" an Mäusen oder Ratten wirkt (Methode vgl. Siegmund et. al., Proc. Soc. Exp. Biol. 95, (1957), 729-731). Die analgetische Wirkung als solche läßt sich ferner im "Tail-Flick-Test" an Mäusen oder Ratten nachweisen (Methodik vgl. d'Amour and Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur). Besonders starke Wirkungen sind an Ratten im Modell der Carrageenin-induzierten Hyperalgesie (vgl. Bartoszyk und Wild, Neuroscience Letters 101 (1989) 95) zu beobachten. Dabei zeigt die Verbindung keine oder nur geringe Neigung zu physischer Abhängigkeit.

Außerdem wurden durch entsprechende nach geläufigen Methoden durchgeführte Versuche ausgeprägte antiinflammatorische, diuretische, antikonvulsive, neuroprotektive Wirkungen nachgewiesen. Die Verbindung zeigt eine hohe Affinität in bezug auf das Bindungsverhalten an kappa-Rezeptoren.

EMD 61753 ist im Gegensatz zu anderen Verbindungen mit ähnlichem Wirkungsspektrum besonders geeignet für die Verwendung in pharmazeutischen Zubereitungen zur Behandlung entzündlicher Darmerkrankungen, da sie neben der analgetischen und antiinflammatorischen Wirkung geeignet ist, durch die Erkrankung hervorgerufene Störungen der Darmmotorik zu normalisieren. Insbesondere ist sie geeignet, die Darmbewegungen wieder in Gang zu bringen, wenn durch die entzündliche Darmerkrankung ein Darmverschluß droht oder bereits eingetreten ist. Auch kann diese Wirkung zur Behandlung eines postoperativen lleus und der damit verbundenen Schmerzen eingesetzt werden.

In allen hier beschriebenen Indikationsgebieten hat sich die Verwendung von N-Methyl-N-[(1S-)-1-phenyl-2-((3S)-3-hydroxypyrrolidin1-yl-)-ethyl]-2,2-diphenyl-acetamid, Hydrochlorid als Arzneimittel in den verschiedensten Zubereitungsformen als besonders wirksam herausgestellt.

Als besonders vorteilhaft hat sich außerdem bei der Verbindung erwiesen, daß sie aufgrund ihrer Struktur offenbar die Blut-Hirn-Schranke nicht passieren kann und daher kein Abhängigkeitspotential aufweist. Auch wurden bisher keine Wirkungen gefunden, die die Nutzung der vorteilhaften Wirkungen für die beanspruchten Indikationen in irgendeiner Weise einschränken würden.

Im folgenden werden Beispiele gegebenen, die zur Veranschaulichung der Erfindung dienen.

Nachstehend sind alle Temperaturen in ° C angegeben.

### Beispiel 1

### N-Methyl-N-[(1S)-1-phenyl-2-((3S)-3-hydroxypyrrolidin-1-yl)-ethyl]-2,2-diphenylacetamid, Hydrochlorid

In einer 500 ml- Apparatur werden 22 g (2S)-2-N-Carboxyethyl-2-phenylglycin-N,N-[(3S)-3-hydroxy-tetramethylamid] vorgelegt und in 150 ml Tetrahydrofuran gelöst. Unter Rühren wird eine Lösung, bestehend aus 150 ml Tetrahydrofuran und 24,1 g Diphenylacetylchlorid, bei 10 - 20°C innerhalb von einer Stunde zu getropft, wobei zu Beginn sich ein Niederschlag bildet, der jedoch im Verlauf der Reaktion wieder in Lösung geht. Gegen Ende der Reaktion bildet sich erneut ein Niederschlag. Es wird weitere 12 Stunden bei Zimmertemperatur gerührt. Anschließend wird auf ca. 5°C gekühlt und das ausgefallene Produkt abgesaugt. Das abgetrennte Produkt wird mit etwa 100 ml Tetrahydrofuran nachgewaschen und getrocknet. Auf diese Weise werden 39 g Rohprodukt erhalten. Dieses wird mit etwa 250 ml Ethanol und 1 g Aktivkohle umkristallisiert. Ausbeute 33 g (73,2 % der Theorie)

Die pharmazeutische Wirksamkeit der erfindungsgemäßen Substanz in der Behandlung von entzündlichen Darmerkrankungen wurde nach einer in European J. of Pharmacology 271 (1994) 245-251 beschriebenen Methode untersucht. Es wurde die Wirkung von peripher wirkender kappa-Agonisten auf die Aktivität von den Dickdarm versorgenden Nerven, sowohl des gesunden als auch des entzündeten Dickdarms, untersucht. Zu diesem Zweck wurden von insgesamt vierzehn sensorischen Nervenfasern, bei denen es sich bei acht um C-Fasern (langsam leitend) und bei sechs um A-Fasem (schnell leitend) handelte, die Antworten auf cyklische Druckänderungen erfaßt.

Entzündungen im Dickdarmbereich wurden durch Applikation von Trinitrobenzolsulfonsäure hervorgerufen. Die Messungen wurden vier Tage nach der Applikation dieser Substanz durchgeführt.

Die Wirkung von N-Methyl-N-[(1S)-1-phenyl-2-((3S)-3-hydroxypyrrolidin-1-yl)-ethyl]-2,2-diphenylacetamid, Hydrochlorid auf die Nervenentladungen wurde exemplarisch mit der der Standardvergleichssubstanz ICl 204,488 verglichen. N-Methyl-N-[(1S)-1-phenyl-2-((3S)-3-hydroxypyrrolidin-1-yl)-ethyl]-2,2-diphenylacetamid, Hydrochlorid hemmte dosisabhängig die Nervenantwort auf die cyclischen Drucksteigerungen. Im nicht entzündeten Dickdarm wurde die Antwort nach der Gabe von insgesamt 32 mg/kg um 75,4 % gehemmt. Im entzündeten Darm hemmte diese Dosis die Wirkung sogar um 99,8 %. Die ED₅₀-Werte von N-Methyl-N-[(1S)-1-phenyl-2-((3S)-3-hydroxypyrrolidin-1-yl)-ethyl]-2,2-diphenylacetamid, Hydrochlorid betrugen 13 ± 4 mg/kg im nicht entzündeten Dickdarm. ICl 204,488 war dagegen sogar nach der Applikation von insgesamt mg/kg ohne Wirkung.

Die hemmende Wirkung von N-Methyl-N-[(1S)-1-phenyl-2-((3S)-3-hydroxypyrrolidin-1-yl)-ethyl]-2,2-diphenylacetamid, Hydrochlorid wird daher offensichtlich durch die Aktivierung von kappa-opioid-Rezptoren an den sensorischen Nervenenden hervorgerufen, während ICl 204,488 keine Wirkung auf diese Rezeptoren aufweist.

## Patentansprüche

1. Verwendung des Arzneimittels N-Methyl-N-[(1 S)-1-phenyl-2-((3S)-3-hydroxypyrrolidin-1-yl)-ethyl]-2,2-diphenyl-acetamid, Hydrochlorid zur Herstellung von Arzneimittelformulierungen mit der pharmakologischen Wirkung als kappa-Opiatagonist zur Behandlung von entzündlichen Darmerkrankungen.

## Claims

1. Use of the medicament N-methyl-N-[(1S)-1-phenyl-2-((3S)-3-hydroxypyrrolidin-1-yl)ethyl]-2,2-diphenylacetamide, hydrochloride for the production of pharmaceutical formulations with pharmacological action as a kappa-opiate agonist for the treatment of inflammatory bowel disorders.

## Revendications

1. Utilisation du médicament N-méthyle-N-[(1S)-1-phényle-2-((3S)-3-hydroxypyrolidine-1-yl)-éthyle]-2,2-diphénylacétamide chlorhydrique pour la préparation des formulations pharmaceutiques ayant une action pharmacologique comme agoniste kappa opioïde pour le traitement des affections intestinales inflammatoires.
